# EUROPEAN PATENT SPECIFICATION

(11) **EP 4 568 600 B1**
(45) Date of publication and mention of the grant of the patent: **03.12.2025**
(21) Application number: 23751967.3
(22) Date of filing: 03.08.2023
(51) Int. Cl.: A61B 17/80, A61F 2/40, A61B 17/86, A61F 2/30

(54) **SHOULDER PROSTHESIS BASEPLATE WITH COMPRESSIVE SCREW**
BASISPLATTE EINER SCHULTERPROTHESE MIT KOMPRESSIONSSCHRAUBE
PLAQUE DE BASE DE PROTHÈSE D'ÉPAULE AVEC VIS DE COMPRESSION

(30) Priority: 11.08.2022 IT 202200017211
(43) Date of publication of application: 18.06.2025
(73) Proprietor: Limacorporate S.p.A., 33038 San Daniele del Friuli (UD) (IT)
(72) Inventor: FERRO, Thomas, 33050 Mortegliano (UD) (IT); DEL NEGRO, Nicola, 33038 San Daniele del Friuli (UD) (IT)
(74) Representative: Botti & Ferrari S.p.A.
(86) International application number: PCT/EP2023/071576
(87) International publication number: WO 2024/033222

(56) References cited:
- EP-A1- 3 598 957
- WO-A2-2017/007565
- US-A1- 2013 150 901
- US-A1- 2020 289 180

## Description

### Field of Application

The present invention relates to a shoulder prosthesis baseplate comprising a through-hole for a compressive screw. The present invention also relates to a bone anchoring assembly comprising a shoulder prosthesis baseplate and a compressive screw.

### Prior art

The use of a compressive screw is a very common practice in shoulder prosthesis baseplates. The presence of a central compressive screw allows to considerably increase the primary stability of the implant, thus reducing the relative micromovements between the shoulder prosthesis baseplate and the bone interface, so as to reduce the possibility of early mobilization or failure of the implant.

In this context, there is a need to have at disposal compressive screws of different lengths, that are required for proper stability depending on the bone anatomy of the individual patients, and possibly of different diameter.

Although the choice of providing screws of different lengths does not affect the dimensions and the geometry of the shoulder prosthesis baseplate, a difficulty arises when screws of various thread diameters are required. The diameter of the thread of the screw is in fact constrained by the diameter of the through-hole of the shoulder prosthesis baseplate through which the screw must pass.

Screws having threads of different diameter, typically of increased diameter, may be required depending on the bone quality of the individual patient, on the tactile feedback of the surgeon or in case it is decided to use a type of thread for spongy bone with increased screw diameter, rather than a thread for cortical bone.

In the specific case of a shoulder prosthesis baseplate of glenoid prosthesis, the choice of using a central compressive screw having an increased diameter (e.g., Ø6.0 mm or Ø6.5mm) compared to the usual one clashes with the need, which is often required from the perspective of bone conservation and to cope with morphological limitations associated with the glenoid anatomy, of having a baseplate pin of small diameter size.

In this context, the prior art proposes some solutions to use central compressive screws having increased diameter, which are associated with glenoid prosthesis baseplates that are suitably configured.

The document US2020289180A1 refers to a bone anchoring base belonging to a prosthetic joint, having a protrusion. The protrusion comprises an opening at its distal free end, a screw being configured to be engaged through the protrusion and screwed in the spongy portion of a bone, through the opening. The opening is threaded with the same pitch of the thread of the screw, and the diameter of a distal wall is such that it is possible to arrange more than half a turn of the thread into it, so that, during positioning of the screw, the thread of said screw engages with the distal wall.

The document EP3598957A1 refers to a shoulder prosthesis implant comprising a baseplate or metaglene, configured to be connected to a glenosphere. For the insertion of a central bone screw, a through-hole that can accommodate a maximum screw diameter is provided. A pin extends distally and provides a threaded portion configured to support and retain the head of a screw.

The document WO2017/007565A2 refers to a glenoid implant for shoulder prosthesis, which has anchor unit restrained against axial translation with respect to baseplate rotating with respect to unit when external surface of proximal head is fixed proximal of internal surface of aperture.

The prior art solutions that provide a threaded portion in the baseplate, with a real helical fit between screw and baseplate, require that the thread of the exit hole corresponds to the geometry of the thread of the central screw. In fact, the screw screws on the thread made on the pin.

This thread of the exit hole may be difficult to manufacture in a context of production of the specific shoulder prosthesis baseplate.

This thread of the exit hole allows to use only one type of enlarged central screw, having stem thread corresponding to the geometry of the threaded feature. For example, for equal diameters, a different thread pitch could not be used.

Prior art solutions are not completely effective and can therefore be improved.

An object of the present invention is to allow insertion of a compressive screw having an increased diameter into a shoulder prosthesis baseplate, avoiding significantly affecting the dimensions of a hole or of a central pin.

A further object of the present invention is to allow compatibility between a shoulder prosthesis baseplate and further modular products already developed for the same connection area.

A further object of the present invention is to allow a more effective manufacturing in a context of production of a shoulder prosthesis baseplate.

A further object of the present invention is to provide a shoulder prosthesis baseplate adapted to receive more flexibly a variety of screws having different, even increased, threads.

A further object of the present invention is to provide a shoulder prosthesis baseplate having structural and functional features that allow to overcome drawbacks of the prior art.

### Summary of the invention

The present invention is defined in claims 1 and 13 while preferred embodiments are set forth in the dependent claims. A solution idea underlying the present invention is to provide the passage of a compressive screw in an exit hole that has a shaped slot, having only locally a diameter that is greater than the thread that is intended to be passed, whereas in other portions it has a diameter that is less than the external diameter of the thread and such that it retains the head of the compressive screw. In particular, the greater diameter of the slot is a circular arc, preferably less than 180°.

Based on said solution idea, the present invention provides a shoulder prosthesis baseplate comprising a through-hole for a compressive screw. The through-hole has a circular profile section and comprises a projecting edge arranged internally and perpendicularly to an axis of said through-hole. The projecting edge is configured to axially retain a head of the compressive screw inserted in the through-hole. The projecting edge has circular arc shape and delimits a corresponding free portion of the circular profile section internally to the through-hole. The free portion is configured to allow the screwing passage of a threaded stem of the compressive screw to surpass the projecting edge.

Based on said solution idea, the present invention also provides a bone anchoring assembly comprising a shoulder prosthesis baseplate and a compressive screw, which is inserted in the through-hole. The compressive screw comprises a head configured to abut with the projecting edge and thus to be axially retained in the through-hole. Moreover, the compressive screw further comprises a threaded stem having thread diameter and thread pitch such as to allow the screwing passage at the free portion, to surpass the projecting edge of the through-hole.

Advantageously, the present invention does not require any threaded part of the exit hole, but the passage of the compressive screw is based on the presence of a free portion, open along a circular arc sufficient for the passage of the thread.

Preferably, the projecting edge has a substantially planar and non-helical development.

Advantageously, the present invention does not require any helical shaping on the through-hole, but it simply requires a slot that is easier and less complex to make.

In a preferred embodiment, the shoulder prosthesis baseplate has a central pin. When the present invention is applied to a shoulder prosthesis baseplate having a central pin, it has the advantage that it does not change the dimensions of the pin, and moreover it allows to use different types of central compressive screws, even with increased diameter (close to that of the through-hole), without affecting the overall geometry of the shoulder prosthesis baseplate.

Advantageously, the present invention allows to use compressive screws with various thread diameters, even increased diameters, in elements of modular systems of prosthesis having a shoulder prosthesis baseplate, with greater flexibility.

Further features and advantages of the invention will become apparent from the following detailed description of embodiments, which is provided by way of non-limiting illustration, and from the claims which are integral part of the present description.

### Brief description of the drawings

- Figure 1 shows an exemplary embodiment of a shoulder prosthesis baseplate.
- Figure 2 shows a shoulder prosthesis baseplate having a central compressive screw inserted in a through-hole according to a known solution.
- Figure 3 exemplifies the application of the shoulder prosthesis baseplate of Figure 1 to a scapula of a patient.
- Figure 4 shows a sectional view of a shoulder prosthesis baseplate according to the present invention, with a first compressive screw inserted in the through-hole.
- Figure 5 shows a sectional view of the shoulder prosthesis baseplate of Figure 4, with a second, different, compressive screw inserted in the through-hole.
- Figure 6 shows a top view of a shoulder prosthesis baseplate according to the present invention.
- Figure 7 exemplifies a top view of a geometry of a through-hole in a shoulder prosthesis baseplate according to the present invention.
- Figure 8 exemplifies a sectional view of a geometry of a through-hole in a shoulder prosthesis baseplate according to the present invention.
- Figure 9 exemplifies the passage of a compressive screw beyond a projecting edge in a shoulder prosthesis baseplate according to the present invention.
- Figure 10 exemplifies a compressive screw abutting on a projecting edge in a shoulder prosthesis baseplate according to the present invention.
- Figure 11 shows an exemplary embodiment of a shoulder prosthesis baseplate with which a glenoid fixing pin is associated.

In different figures, analogous elements will be indicated by analogous reference numerals. Frequently, if one figure contains more analogous elements, only one or some of them will be indicated by a respective reference numeral for the purpose of improved legibility, construing that also the others are included in the discussion.

### Detailed description

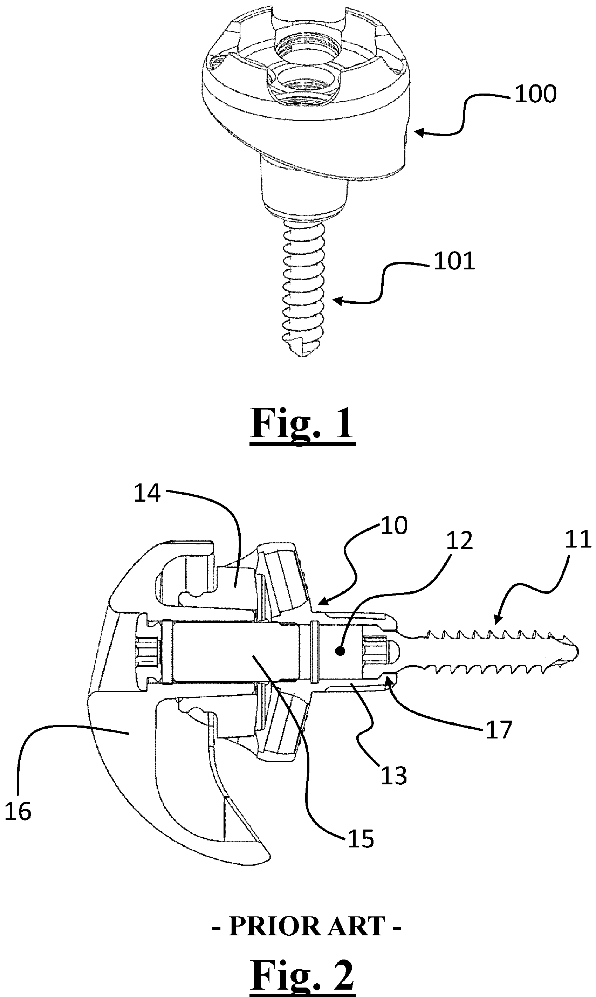

Figure 1 shows an exemplary embodiment of a shoulder prosthesis baseplate 100. The baseplate 100 is for an example of reverse shoulder prosthesis but, in a variant, it could be for an anatomical shoulder prosthesis.

In general, the present invention is applicable to other types of shoulder prosthesis baseplates, such as for example different elements of prosthesis, in particular of modular prostheses, or plates for osteosynthesis. The embodiment of the baseplate 100 to which reference will be made below is thus to be meant as a preferred but non-limiting embodiment.

In general, the shoulder prosthesis baseplate or baseplate 100 comprises a through-hole for a compressive screw 101, that has the function of penetrating a bone surface to provide anchoring, as it will be further described.

Instead, Figure 2 shows a shoulder prosthesis baseplate 10 having a central compressive screw 11 inserted in a through-hole 12, made according to a generic prior art solution, provided herein to better illustrate the technical constraints involved by a possible variation of the thread diameter of a compressive screw, associated with the baseplate 10.

The baseplate 10 and the compressive screw 11 are optimized to maintain small dimensions for the central pin 13 protruding from the baseplate 10. In general, the shoulder prosthesis exemplified herein provides a connector 14, an assembling screw 15 and a glenosphere 16.

Returning to the compressive screw 11, it is noted that the maximum diameter of the thread of the stem and the diameter of the screw head are strictly constrained by the dimension of the circular hole 17 on the pin of the baseplate 10. In fact, to ensure proper passage of the threaded stem of the screw 11, for example having diameter of Ø5.0 mm, a circular hole 17 having a greater diameter, for example having a diameter of Ø5.3 mm, is provided. The head of the screw must have a diameter large enough to be retained by the recess defined at the circular hole 17, for example it must have a Ø6.3 mm diameter. This diameter of the head of the screw 11, in turn, dictates the minimum dimension of the channel defined by the through-hole 12, that can have, for example, a diameter of Ø6.5 mm.

From evident geometrical considerations, it is appreciated that, with the geometry of the baseplate 10 in the example of prior art solution, it is impossible to use a screw of increased dimensions, having a maximum diameter of the thread of the stem that is greater than the diameter of the circular hole 17; for example, it is clearly impossible to implement a threaded stem having a diameter Ø6.0 mm or Ø6.5 mm when the diameter of the circular hole is Ø5.3 mm.

In order to hypothesize a different structure of the baseplate 10 to house screws having threaded stems of diameter Ø6.0 mm, it would be necessary, for example, to accordingly enlarge all the diameters, for example by making a circular hole 17 having a diameter Ø6.4 mm, which would also result in an increase of the external diameter of the pin of the baseplate 10, for example up to Ø10.4 mm, to maintain the minimum thicknesses required for the structural strength of the prosthesis elements.

It is appreciated that a possible enlargement of holes dimensions does not only entail a variation of dimensions (e.g. that can be implemented only on baseplates of large dimensions since the pin has to be enlarged, but it could interfere with lateral holes, especially in the case of baseplates of smaller diameter), but it also affects all the modular components that are subsequently implanted, for example in connection to the assembling screw 15 that has a thread M7 that should be modified in case of enlargement of the pin of the baseplate 10, thereby causing the loss of compatibility of the modular elements among the various versions of the baseplate 10.

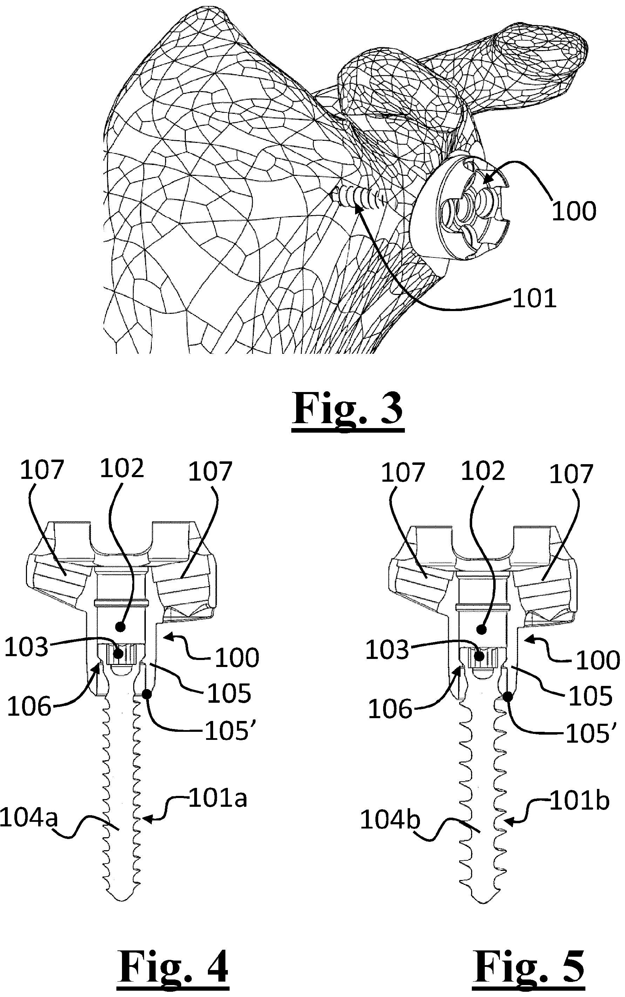

Figure 3 exemplifies the application of the baseplate 100 to a scapula of a patient, by means of the anchorage provided by the compressive screw 101.

As already mentioned, the presence of the central compressive screw 101 allows primary stability of the implant, reducing the relative micromovements between the baseplate 100 and the bone interface.

To obtain a better anchorage, the surgeon may select, also depending on the bone surface available for the individual patient, compressive screws 101 of different length and of different diameter.

Compressive screws 101 having threads of different diameter, typically of increased diameter, are in fact required depending on the bone quality of the individual patient, on the tactile feedback of the surgeon or in case in which it is decided to use a type of thread for spongy bone with increased screw diameter, rather than a thread for cortical bone.

Figure 4 and Figure 5 show sectional views of the same shoulder prosthesis baseplate 100 or baseplate 100, with which a first compressive screw 101a or a second compressive screw 101b, inserted in the through-hole 102, are associated.

In general, it is noted that the axis of the through-hole 102 corresponds to an axis of insertion of the compressive screw 101a or 101b.

Figures 4 and 5, even if they are not exactly drawn to scale, make clear that the first compressive screw 101a has the same diameter of the head 103 with respect to the head 103 of the second compressive screw 101b, but it has a diameter of threaded stem 104a that is less than the diameter of threaded stem 104b of the second compressive screw 101b.

For example, the threaded stem 104a has a maximum diameter of Ø5.0 mm whereas the threaded stem 104b has maximum diameter of Ø6.3 mm.

As mentioned, the baseplate 100 comprises a projecting central pin 105, and the through-hole 102 is also provided inside the central pin 105.

The through-hole 102 has a circular profiles section i.e., is substantially shaped as a circle.

As will be further described, the baseplate 100 comprises a projecting edge 106 inside the through-hole 102. In a preferred embodiment, such projecting edge 106 is placed in an intermediate position inside the through-hole 102, namely axially retracted with respect to an end 105' of the central pin 105. In this way, the pin 105 houses more proximally the head 103 of the screw, which is better protected as well as more accessible from the top of the baseplate 100 during implantation.

Preferably, the baseplate 100 further comprises a plurality of perimeter through-holes 107, that are configured to house additional fixing screws (not shown), in particular angular-stability screws.

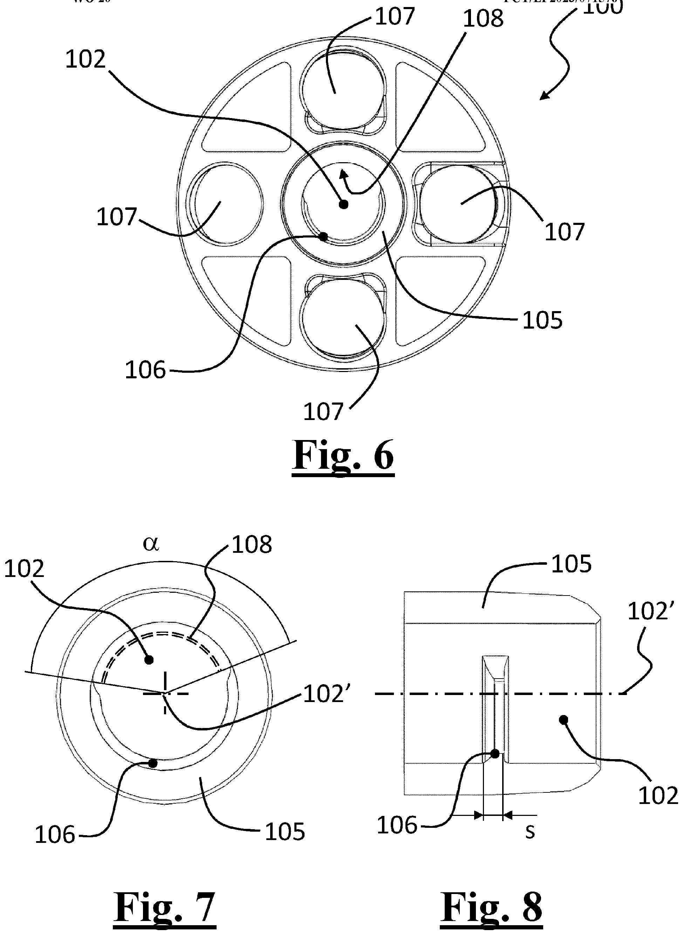

Figure 6 shows a top view of the baseplate 100 in which the through-hole 102 for the compressive screw is clearly visible.

The through-hole 102 comprises a projecting edge 106 arranged internally and perpendicularly to an axis 102' of the through-hole 102.

The projecting edge 106 is configured to axially retain a head 103 of the compressive screw 101 inserted in the through-hole, as already discussed in connection to Figures 4 and 5.

As it can be observed in Figure 6, the projecting edge 106 has circular arc shape i.e., is shaped curved as an arc of a circumference.

Also, the projecting edge 106 delimits a corresponding free portion 108 internally to the through-hole. The corresponding free portion 108 is also located in the circular profile section of the through-hole 102.

As it will be further described, the free portion 108 is configured to allow the screwing passage of a threaded stem 104 of the compressive screw 101 to surpass such projecting edge 106.

Figure 7 shows a top view of the geometry of the through-hole 102, in which the circular arc shape of the projecting edge 106 is visible which, as mentioned, delimits a corresponding free portion 108 internally to said through-hole 102.

The free portion 108 extends in a second circular arc having a central angle α which subtends it. The central angle α has amplitude less than 180°, preferably greater than 90°, more preferably equal to 155°. These values of the central angle α are such as to allow stably housing different screws with different maximum diameters of threaded stem.

Figure 8 exemplifies a sectional view of the geometry of the through-hole 102, in which it can be observed that the projecting edge 106 has a substantially planar and non-helical development, remaining perpendicular to the axis 102' of the through-hole 102.

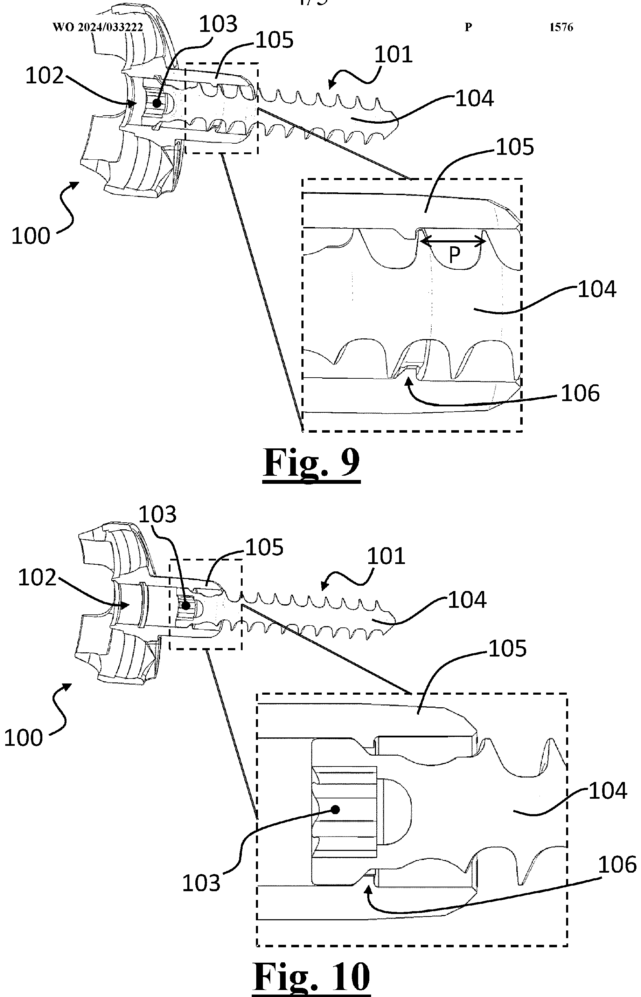

Figure 9 exemplifies the passage of the compressive screw 101 in the through-hole 102, beyond the projecting edge 106. Instead, Figure 10 exemplifies the compressive screw 101 abutting on the projecting edge 106. In such figures, there is an enlarged box that is highlighted by the dashed lines.

In a bone anchoring assembly 100, 101, the already described shoulder prosthesis baseplate 100 or baseplate 100 and an already described compressive screw 101, are provided.

As already mentioned, the compressive screw 101 is inserted in the through-hole 102. Once the head 103 of the screw is completely inserted, it is configured to abut with the projecting edge 106 so as to be axially retained in the through-hole 102.

The compressive screw comprises a threaded stem 104 having thread diameter and thread pitch such as to allow the screwing passage at the free portion 108 defined by the projecting edge 106, so as to surpass the projecting edge 106.

A compressive screw 101 having an increased maximum thread diameter is thus enabled to surpass the projecting edge 106 and to fix into the bone, still remaining axially retained in the through-hole 102 so as to provide anchorage to the baseplate 100.

Very advantageous geometrical proportions of the elements of the baseplate 100 can be defined as follows.

A first internal diameter defined by the projecting edge 106 is less than a second internal diameter defined by the through-hole 102.

A maximum diameter defined by the threaded stem 104 is greater than the first internal diameter defined by the projecting edge 106, and at the same time it is less than a second internal diameter defined by the through-hole 102.

A third diameter defined by the head 103 of the compressive screw 101 is greater than the first internal diameter defined by the projecting edge 106, so that the head 103 is configured to abut onto the projecting edge 106 as shown in Figure 10.

A fourth diameter defined by the free portion 108 is greater than the first internal diameter defined by the projecting edge 106. Preferably, the fourth diameter defined by the free portion 108 corresponds to the second internal diameter defined by the through-hole 102, i.e., the lateral wall of the through-hole 102 has no elevations nor hollows at the free portion 108. It should be clarified that the fourth diameter defined by the free portion 108 is in connection with a circular arc that locally matches the external contour of the free portion 108, outside of which there is the projecting edge 106, as is well visible in Figure 7. An axial thickness S of the projecting edge 106 is less than a minimum or nominal thread pitch P of the threaded stem 104, so as to allow the screwing passage of the threaded stem 104 at the free portion 108, and so as to surpass the projecting edge 106.

Preferably, the projecting edge 106 is tapered to the through-hole 102 at least in the area configured to abut with the head 103 of the compressive screw 101.

In general, for a given compressive screw 101 defined by a thread geometry (thread pitch, thread diameter, core diameter) there are several combinations of increased diameter and thickness of the projecting edge 106 that are compatible with the head 103 of the screw and that allow the compressive screw 101, if subjected to screwing, to pass over the projecting edge 106 while avoiding any interference.

Similarly, for a given projecting edge 106 defined by a geometry (increased diameter, edge thickness, extension of the free portion 108) there are several geometries of thread of compressive screws that are compatible for the screwing passage, passing over the projecting edge 106 while avoiding any interference.

In the specific example of the pin 105, it was possible to develop a compressive screw 101 with threaded stem 104 having maximum diameter of Ø6.3mm and capable of passing inside the through-hole 102 in the free portion 108 having an angle α of 155° and a thickness S of 1.0 mm.

The abutment and the stable axial retention of the head 103 of the screw 101 is generally ensured by the fact that the projecting edge is defined along a circular arc which is subtended by a central angle greater than 180°, in the preferred example equal to 205° (i.e., 360° minus 155°).

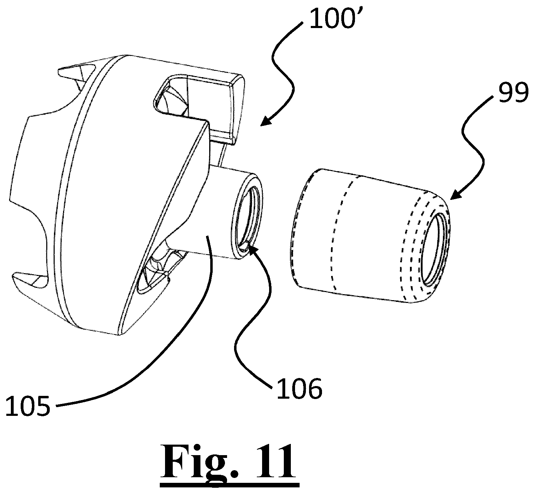

Figure 11 shows an exemplary embodiment of a shoulder prosthesis baseplate 100' or baseplate 100' with which, beside the compressive screw (not shown), a glenoid fixing pin 99 is associable, that is a further element of modular prosthesis. It should be noted that, for the variant of the baseplate 100', the projecting edge 106 is located in a distal position inside the through-hole 102, namely directly at the end of the central pin 105.

It is evident that further implementations and modifications of the present invention will be possible for the person skilled in the art to meet particular needs.

For example, different specific geometries of the compressive screw may be provided, since said compressive screw is not constrained to engage in standardized threaded holes, but it is intended to screw into the bone.

Said different specific geometries will be widely compatible for the screwing passage of the threaded stem to surpass the projecting edge, functional for a plurality of threads.

Therefore, the above-described embodiments are to be understood as provided by way of non-limiting illustration.

## Claims

1. Shoulder prosthesis baseplate (100) comprising a through-hole (102) for a compressive screw (101),
wherein said through-hole (102) has a circular profile section and comprises a projecting edge (106) arranged internally and perpendicularly to an axis (102') of said through-hole (102),
said projecting edge (106) being configured to axially retain a head (103) of said compressive screw (101) inserted in said through-hole (102),
**characterized in that** said projecting edge (106) has circular arc shape and delimits a corresponding free portion (108) of said circular profile section internally to said through-hole (102),
said free portion (108) being configured to allow the screwing passage of a threaded stem (104) of said compressive screw (101) to surpass said projecting edge (106).

2. Shoulder prosthesis baseplate according to claim 1, wherein said projecting edge (106) has a substantially planar and non-helical development.

3. Shoulder prosthesis baseplate according to claim 1 or 2, wherein said free portion (108) extends in a second circular arc subtended by a central angle (α) less than 180°, preferably greater than 90°, more preferably equal to 155°.

4. Shoulder prosthesis baseplate according to any one of claims 1 to 3, wherein a first internal diameter of said projecting edge (106) is less than a second internal diameter of said through-hole (102).

5. Shoulder prosthesis baseplate according to claim 4, wherein a maximum diameter of said threaded stem (104) is greater than said first internal diameter of said projecting edge (106) and less than said second internal diameter of said through-hole (102).

6. Shoulder prosthesis baseplate according to claim 4 or 5, wherein a third diameter of said head (103) is greater than said first internal diameter of said projecting edge (106), said head (103) being configured to abut onto said projecting edge (106).

7. Shoulder prosthesis baseplate according to any one of claims 4 to 6, wherein a fourth diameter of said free portion (108) is greater than said first internal diameter of said projecting edge (106) and preferably corresponds to said second internal diameter of said through-hole (102).

8. Shoulder prosthesis baseplate according to any one of claims 4 to 7, wherein an axial thickness (S) of said projecting edge (106) is less than a thread pitch (P) of said threaded stem (104) of said compressive screw (101).

9. Shoulder prosthesis baseplate according to any one of claims 1 to 8, wherein said projecting edge (106) is tapered to said through-hole (102) at least in an area configured to abut with said head (103) of said compressive screw (101).

10. Shoulder prosthesis baseplate according to any one of claims 1 to 9, comprising a central pin (105) projecting from said shoulder prosthesis baseplate, said through-hole (102) being provided inside said central pin.

11. Shoulder prosthesis baseplate according to claim 10, wherein said projecting edge (106) is placed in an intermediate position in said through-hole (102), axially retracted with respect to an end (105') of said central pin (105).

12. Shoulder prosthesis baseplate according to any one of claims 1 to 11, wherein said shoulder prosthesis baseplate further comprises a plurality of perimeter through-holes (107), preferably configured to house angular-stability fixing screws.

13. Bone anchoring assembly comprising a shoulder prosthesis baseplate (100) according to any one of claims 1 to 12 and further comprising a compressive screw (101) inserted in said through-hole (102), said compressive screw (101) comprising a head (103) configured to abut with said projecting edge (106), said head (103) being axially retained in said through-hole (102), said compressive screw (101) further comprising a threaded stem (104) having thread diameter and thread pitch (P) such as to allow the screwing passage at said free portion (108), to surpass said projecting edge (106).

## Patentansprüche

1. Schulterprothesen-Basisplatte (100), die ein Durchgangsloch (102) für eine Kompressionsschraube (101) umfasst,
wobei das Durchgangsloch (102) ein kreisförmiges Profilsegment hat und eine vorstehende Kante (106) aufweist, die innen und senkrecht zur Achse (102') des Durchdurchgangslochs (102) angeordnet ist,
wobei die vorstehende Kante (106) so konfiguriert ist, dass sie einen Kopf (103) der Kompressionsschraube (101), die in das Durchgangsloch (102) eingesetzt ist, axial hält,
**dadurch gekennzeichnet, dass** die vorstehende Kante (106) eine Kreisbogenform hat und einen entsprechenden freien Abschnitt (108) des kreisförmigen Profilsegments im Durchgangsloch (102) begrenzt,
wobei der freie Abschnitt (108) so konfiguriert ist, dass der Gewindeschaft (104) der Kompressionsschraube (101) beim Durchgang während des Einschraubens an dieser vorstehenden Kante (106) vorbei gelangt.

2. Schulterprothesen-Basisplatte nach Anspruch 1, wobei die vorstehende Kante (106) einen im Wesentlichen flachen und nicht schraubenlinienförmigen Verlauf hat.

3. Schulterprothesen-Basisplatte nach Anspruch 1 oder 2, wobei der freie Abschnitt (108) in einem zweiten Kreisbogen verläuft, der unter einem zentralen Winkel (α) von weniger als 180°, vorzugsweise von mehr als 90°, stärker bevorzugt von 155° geschnitten wird.

4. Schulterprothesen-Basisplatte nach einem der Ansprüche 1 bis 3, wobei ein erster Innendurchmesser der vorstehenden Kante (106) kleiner als ein zweiter Innendurchmesser des Durchgangslochs (102) ist.

5. Schulterprothesen-Basisplatte nach Anspruch 4, wobei ein maximaler Durchmesser des Gewindeschafts (104) größer als der erste Innendurchmesser der vorstehenden Kante (106) und kleiner als der zweite Innendurchmesser des Durchgangslochs (102) ist.

6. Schulterprothesen-Basisplatte nach Anspruch 4 oder 5, wobei ein dritter Durchmesser des Kopfs (103) größer als der erste Innendurchmesser der vorstehenden Kante (106) ist, wobei der Kopf (103) so konfiguriert ist, dass er an der vorstehenden Kante (106) anliegt.

7. Schulterprothesen-Basisplatte nach einem der Ansprüche 4 bis 6, wobei ein vierter Durchmesser des freien Abschnitts (108) größer als der erste Innendurchmesser der vorstehenden Kante (106) ist und vorzugsweise einem zweiten Innendurchmesser des Durchdurchgangslochs (102) entspricht.

8. Schulterprothesen-Basisplatte nach einem der Ansprüche 4 bis 7, wobei eine axiale Dicke (S) der vorstehenden Kante (106) kleiner als eine Gewindesteigung (P) des Gewindeschafts (104) der Kompressionsschraube (101) ist.

9. Schulterprothesen-Basisplatte nach einem der Ansprüche 1 bis 8, wobei die vorstehende Kante (106) am Durchgangsloch (102) wenigstens in einem Bereich, der so konfiguriert ist, dass er am Kopf (103) der Kompressionsschraube (101) anliegt, konisch zulaufend ist.

10. Schulterprothesen-Basisplatte nach einem der Ansprüche 1 bis 9, die einen zentralen Stift (105) umfasst, der von der Schulterprothesen-Basisplatte vorsteht, wobei das Durchgangsloch (102) im zentralen Stift vorgesehen ist.

11. Schulterprothesen-Basisplatte nach Anspruch 10, wobei die vorstehende Kante (106) an einer mittleren Position im Durchgangsloch (102) angeordnet ist, wobei sie in Bezug auf ein Ende (105') des zentralen Stifts (105) axial zurückversetzt ist.

12. Schulterprothesen-Basisplatte nach einem der Ansprüche 1 bis 11, wobei die Schulterprothesen-Basisplatte ferner mehrere Umfangsdurchgangslöcher (107) umfasst, die vorzugsweise konfiguriert sind, Fixierschrauben für eine Winkelstabilität aufzunehmen.

13. Knochenverankerungsanordnung, die eine Schulterprothesen-Basisplatte (100) nach einem der Ansprüche 1 bis 12 umfasst und ferner eine Kompressionsschraube (101), die in das Durchgangsloch (102) eingesetzt wird, umfasst, wobei die Kompressionsschraube (101) einen Kopf (103) umfasst, der so konfiguriert ist, dass er an der vorstehenden Kante (106) anliegt, wobei der Kopf (103) im Durchgangsloch (102) axial gehalten wird, wobei die Kompressionsschraube (101) ferner einen Gewindeschaft (104) umfasst, der einen Gewindedurchmesser und eine Gewindesteigung (P) aufweist, so dass er beim Durchgang während des Einschraubens am freien Abschnitt (108) der vorstehenden Kante (106) vorbei gelangen kann.

## Revendications

1. Plaque de base pour prothèse d'épaule (100) comprenant un trous passant (102) pour une vis de compression (101),
dans laquelle ledit trous passant (102) a une section de profil circulaire et comprend un bord saillant (106) disposé à l'intérieur et perpendiculairement à un axe (102') dudit trous passant (102),
ledit bord saillant (106) étant configuré pour retenir axialement une tête (103) de ladite vis de compression (101) insérée dans ledit trous passant (102),
**caractérisée en ce que** ledit bord saillant (106) a une forme d'arc circulaire et délimite une portion libre correspondante (108) de ladite section de profil circulaire à l'intérieur dudit trous passant (102),
ladite portion libre (108) étant configurée pour permettre le passage en vissage d'une tige filetée (104) de ladite vis de compression (101) au-delà dudit bord saillant (106).

2. Plaque de base pour prothèse d'épaule selon la revendication 1, dans laquelle ledit bord saillant (106) a un développement sensiblement planaire et non hélicoïdal.

3. Plaque de base pour prothèse d'épaule selon la revendication 1 ou 2, dans laquelle ladite portion libre (108) s'étend selon un deuxième arc circulaire sous-tendu par un angle au centre (α) inférieur à 180°, préférablement supérieur à 90°, plus préférablement égal à 155°.

4. Plaque de base pour prothèse d'épaule selon l'une quelconque des revendications 1-3, dans laquelle un premier diamètre interne dudit bord saillant (106) est inférieur à un deuxième diamètre interne dudit trous passant (102).

5. Plaque de base pour prothèse d'épaule selon la revendication 4, dans laquelle un diamètre maximal de ladite tige filetée (104) est supérieur audit premier diamètre interne dudit bord saillant (106) et inférieur audit deuxième diamètre interne dudit trous passant (102).

6. Plaque de base pour prothèse d'épaule selon la revendication 4 ou 5, dans laquelle un troisième diamètre de ladite tête (103) est supérieur audit premier diamètre interne dudit bord saillant (106), ladite tête (103) étant configurée pour bouter sur ledit bord saillant (106).

7. Plaque de base pour prothèse d'épaule selon l'une quelconque des revendications 4-6, dans laquelle un quatrième diamètre de ladite portion libre (108) est supérieur audit premier diamètre interne dudit bord saillant (106) et correspond préférablement audit deuxième diamètre interne dudit trous passant (102).

8. Plaque de base pour prothèse d'épaule selon l'une quelconque des revendications 4-7, dans laquelle une épaisseur axiale (S) dudit bord saillant (106) est inférieure à un pas de filetage (P) de ladite tige filetée (104) de ladite vis de compression (101).

9. Plaque de base pour prothèse d'épaule selon l'une quelconque des revendications 1-8, dans laquelle ledit bord saillant (106) est effilé vers ledit trous passant (102) au moins dans une zone configurée pour buter contre ladite tête (103) de ladite vis de compression (101).

10. Plaque de base pour prothèse d'épaule selon l'une quelconque des revendications 1-9, comprenant une broche centrale (105) saillante depuis ladite plaque de base de la prothèse d'épaule, ledit trous passant (102) étant prévu à l'intérieur de ladite broche centrale.

11. Plaque de base pour prothèse d'épaule selon la revendication 10, dans laquelle ledit bord saillant (106) est placé dans une position intermédiaire dans ledit trous passant (102), axialement rétracté par rapport à une extrémité (105') dudit broche central (105).

12. Plaque de base pour prothèse d'épaule selon l'une quelconque des revendications 1-11, dans laquelle ladite plaque de base de prothèse d'épaule comprend en outre une pluralité de trous passants périmétriques (107), préférablement configurés pour loger des vis de fixation de stabilité angulaire.

13. Ensemble d'ancrage osseux comprenant une plaque de base de prothèse d'épaule (100) selon l'une quelconque des revendications 1-12 et comprenant en outre une vis de compression (101) insérée dans ledit trous passant (102), ladite vis de compression (101) comprenant une tête (103) configurée pour buter contre ledit bord saillant (106), ladite tête (103) étant retenue axialement dans ledit trous passant (102), ladite vis de compression (101) comprenant en outre une tige filetée (104) ayant un diamètre de filetage et un pas de filetage (P) tels qu'ils permettent le passage en vissage au niveau de ladite portion libre (108), pour dépasser ledit bord saillant (106).
